# EUROPEAN PATENT SPECIFICATION

(11) **EP 1 934 351 B1**
(45) Date of publication and mention of the grant of the patent: **25.05.2011**
(21) Application number: 06809940.7
(22) Date of filing: 07.09.2006
(51) Int. Cl.: C07K 14/415, C12N 15/82, A01H 5/00

(54) **ABIOTIC STRESS TOLERANT GENE FROM AVICENNIA MARINA ENCODING A PROTEIN**
FÜR EIN PROTEIN CODIERENDES ABIOTISCHES STRESSTOLERANZGEN AUS AVICENNIA MARINA
GÈNE TOLÉRANT AU STRESS ABIOTIQUE ISSU DE AVICENNIA MARINA CODANT UNE PROTÉINE

(30) Priority: 09.09.2005 IN CH12612005
(43) Date of publication of application: 25.06.2008
(73) Proprietor: M.S. Swaminathan Research Foundation, Taramani, Chennai 600 113 (IN)
(72) Inventor: PARIDA, Ajay, Chennai 600 113 (IN); VENKATRAMAN, Gayatri, Chennai 600 113 (IN)
(74) Representative: Huenges, Martin
(86) International application number: PCT/IN2006/000339
(87) International publication number: WO 2007/029270

(56) References cited:
- WO-A-03/031631
- DATABASE EMBL [Online] 9 December 2001 (2001-12-09), "900254 Avicennia marina leaf cDNA Library Avicennia marina cDNA clone Am900254 5' similar to putative low temperature and salt responsive protein (AC082644) of Oryza sativa, mRNA sequence." XP002512825 retrieved from EBI accession no. EMBL:BM172899 Database accession no. BM172899
- DATABASE UniProt [Online] 16 August 2004 (2004-08-16), "SubName: Full=Clt1;" XP002512826 retrieved from EBI accession no. UNIPROT:Q6EJA5 Database accession no. Q6EJA5 & JIA Y ET AL: "Cloning and sequence analysis of a low temperature-induced gene from trifoliate orange with unusual pre-mRNA processing" PLANT CELL REPORTS, vol. 23, no. 3, September 2004 (2004-09), pages 159-166, ISSN: 0721-7714
- DATABASE UniProt [Online] 1 March 2003 (2003-03-01), "RecName: Full=Hydrophobic protein LTI6A; AltName: Full=Low temperature-induced protein 6A;" XP002512827 retrieved from EBI accession no. UNIPROT:Q8H5T6 Database accession no. Q8H5T6 & MORSY M R ET AL: "The OsLti6 genes encoding low-molecular-weight membrane proteins are differentially expressed in rice cultivars with contrasting sensitivity to low temperature" GENE, ELSEVIER, AMSTERDAM, NL, vol. 344, 3 January 2005 (2005-01-03), pages 171-180, XP025394815 ISSN: 0378-1119 [retrieved on 2005-01-03]
- DATABASE UniProt [Online] 1 February 2005 (2005-02-01), "SubName: Full=Low temperature and salt responsive protein;" XP002512828 retrieved from EBI accession no. UNIPROT:Q5MJ27 Database accession no. Q5MJ27
- CAPEL JUAN ET AL: "Two homologous low-temperature-inducible genes from Arabidopsis encode highly hydrophobic proteins" PLANT PHYSIOLOGY (ROCKVILLE), vol. 115, no. 2, 1997, pages 569-576, XP002512822 ISSN: 0032-0889
- MEDINA JOAQUIN ET AL: "Developmental and stress regulation of RCI2A and RCI2B, two cold-inducible genes of Arabidopsis encoding highly conserved hydrophobic proteins" PLANT PHYSIOLOGY (ROCKVILLE), vol. 125, no. 4, April 2001 (2001-04), pages 1655-1666, XP002512823 ISSN: 0032-0889
- NYLANDER MARIA ET AL: "The low-temperature- and salt-induced RCI2A gene of Arabidopsis complements the sodium sensitivity caused by a deletion of the homologous yeast gene SNA1" PLANT MOLECULAR BIOLOGY, vol. 45, no. 3, February 2001 (2001-02), pages 341-352, XP002512824 ISSN: 0167-4412
- MEHTA P.A. ET AL.: 'Generation and analysis of expressed sequence tags from the salt-tolerant mangrove species Avicennia marina (Forsk) Vierh' THEORETICAL AND APPLIED GENETICS vol. 110, no. 3, February 2005, pages 416 - 424, XP019321812
- DATABASE MEDLINE [Online] ZHOU H.T. AND LIN P.: 'Extraction of the salt-tolerant cDNA in mangrove Avicennia marina by mRNA differntial display', XP003013085 Database accession no. (NLM11977600) & SHENG WU GONG CHENG XUE BAO vol. 18, no. 1, January 2002, pages 51 - 54
- 'M.S. Swaminathan Research Foundation' FIFTEENTH ANNUAL REPORT OF THE CENTRE FOR RESEARCH ON SUSTAINABLE AGRICULTURAL AND RURAL DEVELOPMENT, 2004-2005, CENTRE FOR RESEARCH ON SUSTAINABLE AGRICULTURAL AND RURAL DEVELOPMENT, TARAMANI CHENNAI 600 113, INDIA 03 June 2006, XP003013086

### SEQUENCE LISTING

<110> MS Swaminathan Research Foundation
   Parida, Ajay Kumar
   Venkatraman, Gayatri
<120> Abiotic stress tolerant gene from Avicennia marina encoding a protein involved in salt tolerance
<130> PCT0044
<150> 1261/CHE/2005
   <151> 2005-09-09
<160> 12
<170> PatentIn version 3.3
<210> 1
   <211> 600
   <212> DNA
   <213> Artificial sequence
<220>
   <223> synthetic sequence
<400> 1
   ggtagagaaa agagagataa tcttgattct gtttaattta ttttgtcttg gtaaaatcaa      60
   acaagggaag gcaaaaatgg ctgaagggac agcaacttgt atcgatattg ttgtggcaat     120
   tcttctgcct ccacttggtg tcttcctcaa gtatggctgc aagggtgaat tctggatttg     180
   tctgctactg accatccttg gttatattcc tggaattatc tatgctgttt gggccatcac     240
   cagggactga tatgttttcc ttgagaaggt atcctattcg attgtcaggt gaagatgcaa     300
   agggccataa agattgcagc tttctttagc acacagacag tgctgatttg ctggtgttag     360
   gctgcaagtc cctttctctt ccacttttgc ttgtaccccc ttttccaatg tctcagatca     420
   ctacttgatt tgatttgatt tgattttatc ttgtcttatt ttgtatttaa tgtgtgatta     480
   aagtgattta tcttagagtg taagtcttgg tttggcccca ctgtatatgc ccctttagta     540
   atgtaagtat ggtaaccgtg aggtactact tactctattt aaaaaaaaaa aaaaaaaaaa     600
<210> 2
   <211> 174
   <212> DNA
   <213> Artificial sequence
<220>
   <223> synthetic sequence
<400> 2
   atggctgaag ggacagcaac ttgtatcgat attgttgtgg caattcttct gcctccactt      60
   ggtgtcttcc tcaagtatgg ctgcaagggt gaattctgga tttgtctgct actgaccatc     120
   cttggttata ttcctggaat tatctatgct gtttgggcca tcaccaggga ctga           174
<210> 3
   <211> 57
   <212> PRT
   <213> Avicennia marina
<400> 3
   Met Ala Glu Gly Thr Ala Thr Cys Ile Asp Ile Val Val Ala Ile Leu
   1               5                   10                  15
   Leu Pro Pro Leu Gly Val Phe Leu Lys Tyr Gly Cys Lys Gly Glu Phe
               20                  25                  30
   Trp Ile Cys Leu Leu Leu Thr Ile Leu Gly Tyr Ile Pro Gly Ile Ile
           35                  40                  45
   Tyr Ala Val Trp Ala Ile Thr Arg Asp
       50                  55
<210> 4
   <211> 42
   <212> DNA
   <213> Artificial sequence
<220>
   <223> synthetic sequence
<400> 4
   gctgccgcag ctgcagccgc tatggctgaa gggacagcaa ct 42
<210> 5
   <211> 54
   <212> DNA
   <213> Artificial sequence
<220>
   <223> Synthetic sequence
<400> 5
   agcggctgca gctgcggcag ctgcggctgc tttgtatagt tcatccatgc catg 54
<210> 6
   <211> 35
   <212> DNA
   <213> Artificial sequence
<220>
   <223> synthetic sequence
<400> 6
   ctagtctaga atgagtaaag gagaagaact tttca 35
<210> 7
   <211> 36
   <212> DNA
   <213> Artificial sequence
<220>
   <223> synthetic sequence
<400> 7
   ccgctcgagt tatttgtata gttcatccat gccatg 36
<210> 8
   <211> 28
   <212> DNA
   <213> Artificial sequence
<220>
   <223> synthetic sequence
<400> 8
   cgcggatcct cagtccctgg tgatggcc 28
<210> 9
   <211> 64
   <212> DNA
   <213> Artificial sequence
<220>
   <223> synthetic Sequence
<400> 9
   gaacaaaagt tgatttctga agaagatctg atggctgaag ggacagcaac ttgtatcgat 60
   attg 64
<210> 10
   <211> 65
   <212> DNA
   <213> Artificial sequence
<220>
   <223> Synthetic sequence
<400> 10
   cagatcttct tcagaaatca acttttgttc catttttgcc ttcccttgtt tgattttacc 60
   aagac 65
<210> 11
   <211> 22
   <212> DNA
   <213> Artificial sequence
<220>
   <223> Synthetic Sequence
<400> 11
   agcggataac atttcacaca gg 22
<210> 12
   <211> 21
   <212> DNA
   <213> Artificial sequence
<220>
   <223> Synthetic Sequence
<400> 12
   gatacaagtt gctgtccctt g 21

## Description

### FIELD OF INVENTION

The present invention relates to transgenic plants exhibiting enhanced tolerance to drought, salt and dehydration. In particular, the transgenic plant comprises an abiotic stress tolerant Am244 DNA derived from *Avicennia marina.*

### BACKGROUND

Environmental factors such as drought, extreme temperatures, high or fluctuating salinity can affect plant growth and performance and in the case of agronomically important plants this may translate to reduce yield. Increasing soil salinization in irrigated areas has necessitated the identification of crop traits or genes, which confer resistance to salinity, either by conventional breeding or through molecular biology techniques (Munns et al. 2002; Cushman and Bohnert 2000). Hyperosmotic stress, such as that caused by exposure of cells to high concentrations of NaCl causes imbalance of cellular ions, change in turgor pressure and cell volume and alters the activity and stability of macromolecules. Although the basic cellular responses appear to be conserved among all plants, plant species employ a variety of mechanisms to cope with osmotic stress. While extensive work on salinity tolerance in Arabidopsis and Mesembryanthemum has led to the identification of candidate salinity sensitive determinants, these plants are not true halophytes (Zhu 2002; Chauhan et al. 2000).

Further, WO 03/031631 describes salt-inducible and salt-responsive nucleic acids from ryegrass and their use methods for the modification of plant responses to *inter alia* salt stress.

Mangroves are facultative halophytes and exclude most of the salt in seawater. In addition, some species such as *A. marina* actively secrete salt. *Avicennia* is a monotypic pantropical mangrove genus with eight species of which *A. marina* is widely distributed both latitudinally and longitudinally. The high salt tolerance of *A. marina* is a consequence of water use efficiency which balances the relation between carbon gains, water loss and ion uptake with the transpiration stream on a low but constant level. *A. marina* grows in coastal regions where the salt concentration can be as high as 9% (Rao 1987). Regulation of inorganic ions occurs partially by exclusion at the roots and also by excretion via salt glands, the excretion rate for sodium and chloride ions being 0.4 and 0.046 µmol m-2 s-1 (Shimony et al. 1973; Boon and Allaway 1982). It is thus an ideal candidate plant for identifying genes conferring salt and drought tolerance.

An *Avicennia marina* cDNA clone which is similar to putative low temperature and salt responsive protein of *Oryza saliva* is disclosed under EMBL Accession Number BM172899.

Further, Preeti A.M. et al. (2005) describe the generation of a cDNA library of *Avicennia marina* genes and its analyses.

In addition, isolation of RNAs of the leaves of *Avicennia marina* cultured under salinity conditions and their analyses by mRNA differential display analysis has been disclosed by Zhou, Lin (2002).

### SUMMARY OF INVENTION

The present invention relates to a transgenic plant comprising Am244 DNA from *Avicennia marina* and exhibiting enhanced tolerance to drought, salt and dehydration. The disclosure also relates to isolation and characterization of cDNA corresponding to abiotic stress tolerant gene (Am244 gene) derived from *Avicennia marina.* Further the invention also provides a method as defined by the claims for producing drought stress, salt stress and/or dehydration stress tolerant transgenic plants.

One aspect of the invention relates to the use of an isolated nucleic acid molecule for enhancing the tolerance to drought stress, salt stress and/or dehydration stress in plants wherein said isolated nucleic acid molecule has a nucleotide sequence with at least 90% homology to the nucleotide sequence set forth in SEQ ID NO: 1 or SEQ ID NO: 2, wherein said sequence codes for a polypeptide having an amino acid sequence as shown in SEQ ID NO: 3.

Another aspect of the invention relates to the use of an isolated nucleic acid molecule for enhancing the tolerance to drought stress, salt stress and/or dehydration stress in plants, wherein said nucleic acid molecule comprises a nucleotide sequence as shown in SEQ ID NO: 1, or SEQ ID NO: 2.

Another aspect of the invention is the use of a polypeptide having an amino acid sequence as shown in SEQ ID NO: 3 for enhancing the tolerance to drought stress, salt stress and/or dehydration stress in plants, wherein said polypeptide is encoded by the nucleic acid molecule having polynucleotide sequence with at least 90% homology to the nucleotide sequence as shown in SEQ ID NO: 1 or SEQ ID NO: 2.

Yet another aspect of the invention provides the use of an expression cassette for enhancing the tolerance to drought stress, salt stress and/or dehydration stress in plants wherein said expression cassette comprises the nucleic acid molecule having polynucleotide sequence with at least 90% homology to the nucleotide sequence as shown in SEQ ID NO: 1 or SEQ ID NO: 2 operably linked to a plant expressible regulatory sequence.

Further the invention also provides the use of a recombinant vector for enhancing the tolerance to drought stress, salt stress and/or dehydration stress in plants comprising the DNA construct comprising the expression cassette, wherein said expression cassette comprises the nucleic acid molecule having polynucleotide sequence with at least 90% homology to the nucleotide sequence as shown in SEQ ID NO: 1 or SEQ ID NO: 2 operably linked to a plant expressible regulatory sequence.

Yet another aspect of the invention provides the use of a recombinant host cell for enhancing the tolerance to drought stress, salt stress and/or dehydration stress in plants, wherein said host cell comprises the recombinant vector wherein the recombinant vector comprises the DNA construct comprising the expression cassette, said expression cassette comprises the nucleic acid molecule having polynucleotide sequence with at least 90% homology to the nucleotide sequence as shown in SEQ ID NO:1 or SEQ ID NO:2 operably linked to a plant expressible regulatory sequence.

Further, the present invention relates to a drought stress, salt stress and/or dehydration stress tolerant transgenic plant or plant cell or plant tissue comprising an expression cassette comprising an isolated nucleic acid molecule having a nucleotide sequence with at least 90% homology to the nucleotide sequence set forth in SEQ ID NO: 1 or SEQ ID NO: 2, wherein said sequence codes for a polypeptide having an amino acid sequence as shown in SEQ ID NO: 3 operably linked to a plant expressible regulatory sequence, wherein the expression of the said nucleic acid molecule results in the enhanced tolerance to drought stress, salt stress and/or dehydration stress in said plant, plant cell and plant tissue.

A further aspect of the invention is a method of producing a drought stress, salt stress and/or dehydration stress tolerant transgenic plant, said method comprising introducing an expression cassette comprising an isolated nucleic acid molecule having a nucleotide sequence with at least 90% homology to the nucleotide sequence set forth in SEQ ID NO: 1 or SEQ ID NO: 2, wherein said sequence codes for a polypeptide having an amino acid sequence as shown in SEQ ID NO: 3 operably linked to a plant expressible regulatory sequence in a plant genome by using a transformation method, thereby producing a drought stress, salt stress and/or dehydration stress tolerant transgenic plant.

### BRIEF DESCRIPTION OF ACCOMPANYING DRAWINGS

FIG 1a: Expression pattern of the Am244 transcript in leaves and roots of *A. marina* seedlings under conditions of salinity stress (500mM NaCl/top panel) and ABA treatment (100µM/bottom panel) at different time intervals.
FIG 1b: Expression pattern of the Am244 transcript in leaves and roots of *A. marina* seedlings under conditions of NaCl (500mM), KCl (500mM) and mannitol (800mM) treatment at different time intervals.
FIG 2: T-DNA segment of the plasmid pGFP-Ala-Am244-C1.
FIG 3: T-DNA segment of the pMyc-Am244-C1.
FIG 4: Guard cells of pGFP-Ala-Am244-C1 transformed Nicotiana tabacum var. Wisconsin 38 tobacco show localization of the green fluorescence at the plasma membrane and close to the cell wall.

### DESCRIPTION OF THE INVENTION

The present invention relates to a transgenic plants comprising *Am244* DNA from *Avicennia marina* and exhibiting enhanced tolerance to drought, salt or dehydration. The disclosure also relates to isolation and characterization of a cDNA corresponding to abiotic stress tolerant gene (Am244 DNA) derived from *Avicennia marina.* Further the invention is also directed to a method as defined in the claims for producing the drought stress, salt stress and/or dehydration stress tolerant transgenic plants.

The specification relates to an isolated nucleic acid molecule for enhanced tolerance to abiotic stress in plant having a nucleotide sequence with at least 90% homology to the nucleotide sequence set forth in SEQ ID NO: I or SEQ ID NO: 2, wherein said sequence codes for a polypeptide having amino acid sequence as shown in SEQ ID NO: 3.

The specification relates to the isolated nucleic acid molecule having a nucleotide sequence as shown in SEQ ID NO: 1.

The specification relates to the isolated nucleic acid molecule having a nucleotide sequence as shown in SEQ ID NO: 2.

The specification is directed to an isolated nucleic acid molecule encoding a polypeptide comprising an amino acid sequence as shown in SEQ ID NO: 3.

The specification relates to a polypeptide having an amino acid sequence as shown in SEQ ID NO: 3, wherein said polypeptide is encoded by the nucleic acid of the present invention.

The specification provides the isolated nucleic acid molecule having nucleotide sequence as shown in SEQ ID NO: 1 and SEQ ID NO: 2 for enhanced tolerance to abiotic stress such as drought stress, salt stress and dehydration stress in plants.

The specification relates to an expression cassette for conferring enhanced tolerance to abiotic stress in a plant, wherein said expression cassette comprises the aforementioned nucleic acid molecule operably linked to a plant expressible regulatory sequence.

The specification provides the regulatory sequence such as CaMV 35S, NOS, OCS, AdhI, AdhII and Ubi-1.

The specification relates to a DNA construct comprising the expression cassette having nucleic acid molecule as set forth in SEQ ID NO: 1 or SEQ ID NO: 2.

Aforementioned DNA construct further comprises another expression cassette comprising a selectable marker gene operably linked to the regulatory sequence.

The selectable marker gene such as *nptII, hptII, pat* and *bar* can be used for the selection of the transformed plant, plant cell and plant tissues thereof.

The specification provides the DNA construct further comprising another expression cassette comprising a scorable marker gene selected from a group consisting of *GUS, GFP, LUC and CAT* operably linked to the regulatory sequence.

The specification discloses a recombinant vector comprising the aforementioned DNA constructs wherein the constructs comprises the nucleic acid molecule having polynucleotide sequence as shown in SEQ ID NO: 1 or SEQ ID NO: 2.

The specification provides the recombinant plant transformation vector comprising the nucleic acid molecule having polynucleotide sequence as shown in SEQ ID NO: 1 or SEQ ID NO: 2.

The specification relates to a recombinant host cell comprising the recombinant vector comprising the nucleic acid molecule having polynucleotide sequence as shown in SEQ ID NO: 1 or SEQ ID NO: 2, wherein the host cell can be prokaryotic or eukaryotic cell such as *E. coli orAgrobacterium* or plant cell.

Various strains of *E. coli* known in the art such as JM101, DH5α, BL21, HB101, and XL1-Blue can be used for the production of recombinant *E. coli* cell comprising the nucleic acid molecule having polynucleotide sequence as shown in SEQ ID NO: 1 or SEQ ID NO: 2.

The specification provides recombinant *Agrobacterium* cells comprising the nucleic acid molecule having polynucleotide sequence as shown in SEQ ID NO: 1 or SEQ ID NO: 2.

Different *Agrobacterium* strains provided in the art for example LBA4404, EHA101, EHA105, GV3101 and A281 may be used for the production of the recombinant *Agrobacterium.*

The specification provides a plant cell comprising the nucleic acid molecule having polynucleotide sequence as shown in SEQ ID NO: 1 or SEQ ID NO: 2.

The specification provides an abiotic stress tolerant transgenic plant or plant cell or plant tissue comprising the disclosed nucleic acid molecule , wherein the expression of the said nucleic acid molecule results in enhanced tolerance to abiotic stress in said plant, plant cell and plant tissue. Further it also provides the progeny derived from the transgenic plants and seeds produced from them.

The specification is directed to a method of producing an abiotic stress tolerant transgenic plant, said method comprising introducing a nucleic acid molecule as shown in SEQ ID NO: 1 or SEQ ID NO: 2 in a plant genome by using a transformation method, thereby producing an abiotic stress tolerant transgenic plant.

The specification relates to the transformation methods used to develop abiotic stress tolerant transgenic plants.

Plant transformation can be carried out by several methods already known in the art such as *Agrobacterium* mediated transformation, particle bombardment, vacuum-infiltration, *in planta* transformation and chemical methods.

The specification is directed to an *Agrobacterium* mediated transformation method for producing an abiotic stress tolerant transgenic plant, said method comprising:
a) obtaining suitable explants from a plant;
b) constructing the recombinant vector as described in the instant invention;
c) mobilizing said vector in an *Agrobacterium* cell to produce a recombinant *Agrobacterium* cell;
d) co-cultivating said explants with said recombinant *Agrobacterium* cell to produce transformed plant cells,
e) culturing said transformed plant cells to produce an abiotic stress-tolerant transgenic plant.

Plants suitable for transformation with the vectors can be a monocotyledonous and dicotyledonous plant. The monocotyledonous plant is selected from a group consisting of rice, maize, wheat, barley and sorghum. Further the monocotyledonous plant is a rice plant. The dicotyledonous plant is selected from a group consisting of tobacco, tomato, pea, soybean, *Brassica,* okra, chickpea and pigeon pea. The dicotyledonous plant is a tobacco plant.

A broad range of other monocotyledonous or a dicotyledonous plants including cereal crops, pulse crops, vegetables, and other crops can also be used.

Examples of the monocotyledonous plant include wheat, rice, barley, maize, oats, millets, sorghum, sugarcane and rye.

Examples of dicotyledonous plant includes pea, chickpea, tobacco, pigeonpea, Arabidopsis, soybean, brinjal, tomato, cucumber, brassicas, cauliflower, cabbage, cotton.

The specification further relates to the explants used for transformation. Further they are selected from a group consisting of cotyledons, hypocotyls, leaves, anthers, callus, cotyledonary nodes, stems and roots.

The abiotic stress tolerant gene designated as *Am244* gene derived from *A. marina,* belongs to the uncharacterized upf0057 family of putative plasma membrane proteins and is found to be strongly upregulated in the present study in response to abiotic stresses. As with Am244, homologous genes identified in other plant species have also been associated with abiotic stress response and this has also been observed for *Saccharomyces.* In the salt stress tolerant *Lophopyrum,* ESI3 was upregulated within 2 hours of treatment with 250 mM NaCl and also by treatment with KCI, ABA and osmotic shock (Gulick et al, 1994). The Phytophthora Ric1 gene has been shown to be induced by extremes of pH as well as NaCl treatment (van West et al, 1999). *Saccharomyces* PMP3 transcript is rapidly and strongly upregulated (17 fold) within 10 minutes of treatment growth in 1M NaCl (Yale and Bohnert, 2001). Further, deletion of the yeast homolog, PMP3 causes salt sensitivity and membrane hyperpolarization and expression of *Arabidopsis* RCI2A cDNA can complement the pmp3 deletion mutant, indicating that the plant and yeast proteins have similar functions during high salt stress.

The present disclosure relates to plant growth conditions and RNA and DNA isolation. Seeds of *A. marina* collected from their natural mangrove habitat Pichavaram, Tamil Nadu, India. Seeds were grown in sand-filled trays in the green house at 37°C and 12 h light/dark photoperiod (illuminated from 06:00 to 18:00) in near-submergence conditions and watered daily.

Leaf tissue was harvested and total RNA was isolated according to the method given by Chomczynski & Sacchi, 987. RNA isolation can also be carried out by other methods known in art. Total mRNA can also be extracted using such known protocols optimized for isolation of plant RNA using TRIZOL method. The RNA isolation from *A. marina* seedling can also be carried out using commercially available plant RNA isolation kits. Details of growth conditions and RNA isolation are given in Example 1.

The present disclosure is directed to a cDNA library construction. The method for synthesis of cDNA and cloning in suitable vectors are well known in art. Several kits are available for cDNA synthesis from (A+) enriched RNA and well known to the person skilled in the art. Kits for cloning cDNA inserts both directionally and randomly are also well known and can be employed. Many kinds of commercially available vectors can alternatively be used for library preparation such as λ gut10 and λgt11. The ligated cDNA library was transformed into *E. coli* DH5α. For further details see Example 2. A library of approximately 10⁵ recombinants was obtained (Parani M, 1999). Plasmid DNA from several randomly selected clones was extracted by alkaline lysis (Feliciello and Chinali 1993). The DNA sequence of the selected clones was determined by using conventional methods of sequencing to generate expressed sequenced tags (ESTs). 10-12 ESTs were randomly selected and analysed for expression (Northern) under salinity stress conditions (0.5M NaCl) in *Avicennia marina.* One of these genes was found to be up-regulated in both leaves and roots of *Avicennia marina* under salinity stress. The clone was designated as Am244 and was selected for further analysis. For details see Example 2.

The polynucleotide sequence of cDNA of *Am244* gene is shown in SEQ ID NO: 1. The cDNA of *Am244* gene is 600 bp in length and encodes a protein consisting of 57 amino acids (SEQ ID NO: 3). The nucleotide sequence ORF of Am244 cDNA is given in SEQ ID NO: 2.

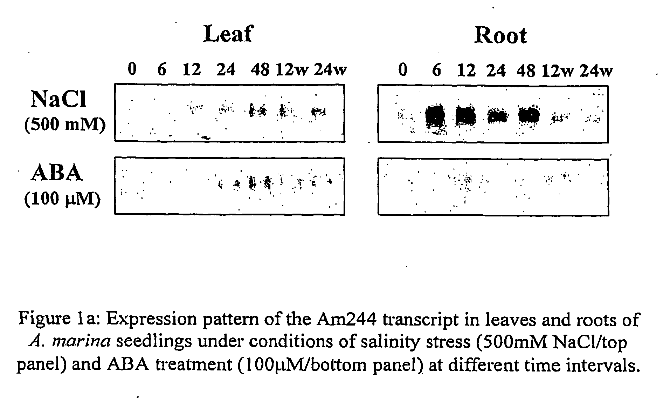

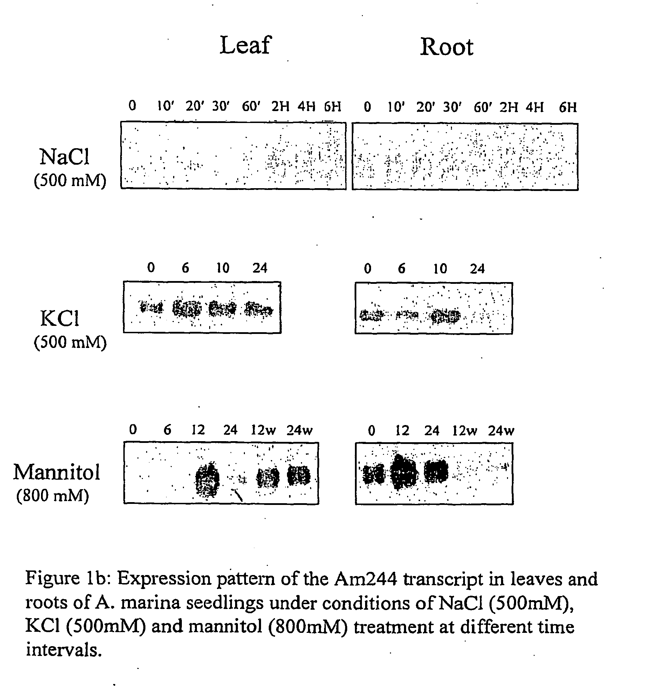

Yet another disclosure relates to the expression analysis of Am244 gene in response to diverse abiotic stresses. Regulation of Am244 gene was analyzed by studying the effect of various abiotic stresses such as NaCl, KCl, ABA and Mannitol. Total RNA was isolated from the plants subjected to various stress condition according to the method given by Chomczynski and Sacchi, 1987 and northern analysis was carried out. Details are provided in Example 3. Am244 was identified to be upregulated for salt, ABA and drought stress. See FIG 1a and 1b.

Additional disclosure provides the BLAST analysis of Am244 protein. The Am244 protein sequence compared with the protein sequences available in various databases for searching the homology with other related protein sequence which show up-regulation in response to abiotic stress conditions. Details are given in Example 4.

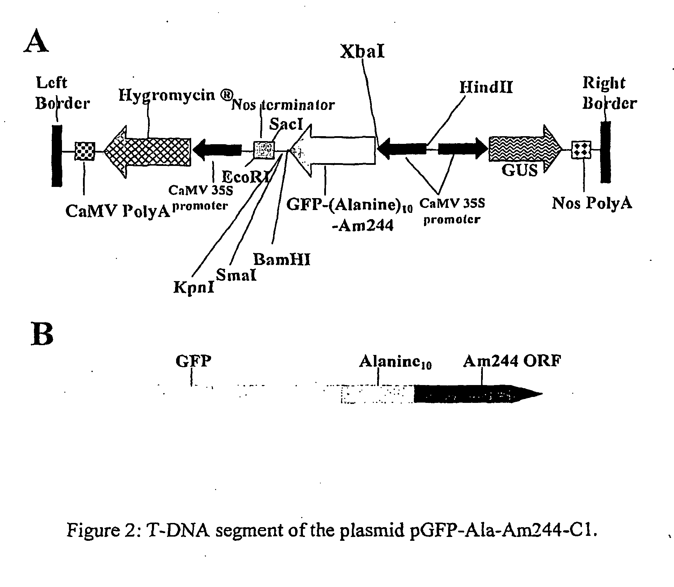

The disclosure is directed towards GFP fusion with Am244 cDNA (GFP-Alanine₁₀-Am244) and construction of plant transformation vector comprising a fragment comprising of GFP (Green Fluorescent protein) with a flexible (Alanine) ₁₀ linker to the N-terminus of the Am244 ORF (SEQ ID NO: 2). This was accomplished by Splice Overlap Extension (SOEing). (See FIG 2). Such expression or recombinant vectors may be constructed by methods known in the art.

Various recombinant vectors comprising GFP gene fused to Am244 ORF having nucleotide sequence as shown in SEQ ID NO: 1 or SEQ ID NO: 2 operably linked to the regulatory sequences such as CaMV 35S, NOS, OCS, AdhI, AdhII and Ubi-1. Details are provided in Example 5.

The disclosure provides epitope tagging of the Am244 gene. The Am244 ORF codes for a small protein of 57 amino acids of which a substantial part is buried in the plasma membrane as two transmembrane domains. Raising antibodies against membrane spanning proteins is not easy. This can be circumvented by epitope tagging of the Am244 ORF.

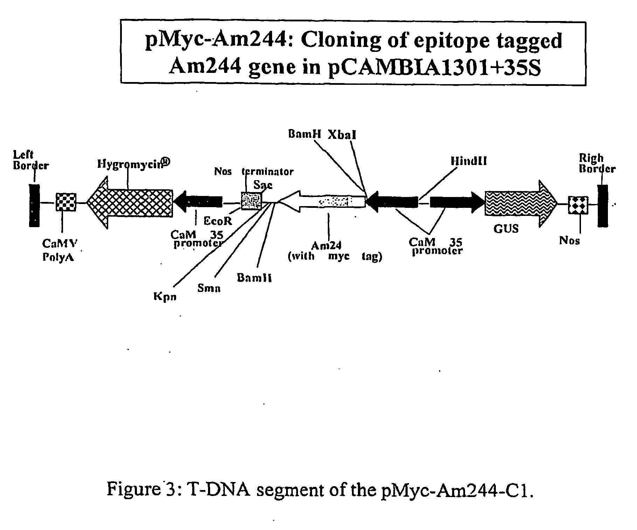

Epitope tagging is a versatile tool used to study proteins, wherein a well characterized peptide tag is fused in-frame with the protein open reading frame (ORF) using recombinant DNA techniques. Epitope tagging can be used to characterize proteins (especially specific members of multigene families), determine subcellular localization, establish topology of membrane proteins, identify interacting partners and track movement within the cell. Epitope-specific commercial antibodies (usually monoclonal) can be then used to address questions about protein localization and function. The c-myc epitope is a well characterized one against which numerous commercially generated monoclonal antibodies are available. The 5' and 3' UTRs of the Am244 cDNA were not disturbed and by insertion mutagenesis using partially complementary primers a c-myc epitope was introduced at the N-terminus of the Am244 ORF. Detailed procedure is given in Example 6. (See FIG 3).

It involves mobilization of various recombinant plant transformation vectors as described above and in the given examples into *Agrobacterium tumefaciens* strain LBA4404 by the freeze-thaw method. Various other strains of *Agrobacterium* known in the art such as EHA101, EHA105, A281 may be used for the transformation. For details see Example 5. Yet another embodiment of the present invention is directed to a method for producing an abiotic stress tolerant transgenic tobacco plant (*Nicotiana tabacum)* cv. Petit Havana expressing Am244 DNA from *A. marina* (See Example 5 and 7 for details).

Other methods known to persons skilled in the art can also be employed for *Agrobacterium transformation.* Apart from freeze thaw method, one may mobilize the vectors into the *Agrobacterium* strain also by electroporation or tri-parental mating. All these techniques are well known in the art.

Similarly, transgenic rice plants expressing the *Am244* gene from *A. marina* were produced by using the recombinant vectors disclosed herein by the transformation method known in the art. Details are given in Example 8.

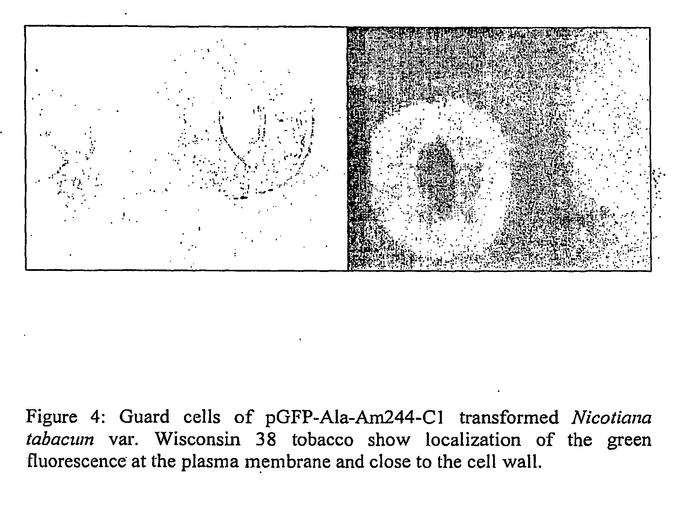

Microscopy and Imaging of the leaf tissue of the transgenic tobacco and rice plant was carried out for the localization of GFP protein in the transgenic plant tissue. Details are given in Example 9. (See FIG 4).

The disclosure provides the screening of presence Am244 DNA in transgenic tobacco and rice by PCR using gene specific primers. Other methods well known to persons skilled in the art can also be employed.

The disclosure relates to confirming the integration of *A₁n244* DNA in single copy in transgenic tobacco and rice by southern hybridization method (Sambrook et al. 2001). For details see Example 7.

Further the disclosure provides the expression analysis of the transgenic tobacco and rice plants subjected to various abiotic stress treatments. For details refer Example 10.

### EXAMPLES

### EXAMPLE 1

### Plant Growth Conditions

Seeds were grown in sand-filled trays in the green house at 37°C and 12 h light/dark photoperiod (illuminated from 6hrs to 18hrs) in near-submergence conditions and watered daily. One-month-old *A. marina* seedlings (four-leaf stage) were acclimatized for 72 hours in 0.5 X Murashige & Skoog (MS) medium (no pH adjustment). Subsequently the plants were transferred into 0.5X MS medium supplemented with 0.5M NaCl for 48 hours.

### RNA Isolation

Leaf and root tissue from plants grown under conditions as mentioned above was harvested and total RNA was isolated according to the method given by Chomczynski and Sacchi, 1987. Leaf and root tissue was harvested from pooled plants and five grams of tissue was macerated in liquid nitrogen and suspended in 18 ml of RNA extraction buffer. To the slurry, 1.8 ml of 2 M sodium acetate (pH 4.0), 18 ml of water saturated phenol and 3.6 ml of 49:1 chloroform: isoamyl alcohol were sequentially added and mixed by inversion. The contents were mixed and cooled on ice for 15 minutes. Finally, the suspension was centrifuged at 10,000 x g for 10 minutes at 4°C. After centrifugation, the aqueous phase was transferred to a fresh tube and mixed with equal volume of ice-cold isopropanol and incubated at -20°C for overnight. The samples were centrifuged at 10,000 x g for 20 minutes at 4°C and the pellet was dissolved in 5 ml of RNA extraction buffer. The RNA was again re-precipitated with equal volume of ice-cold isopropanol. The pellet was washed in 70% ethanol and finally dissolved in formamide. Purity of the RNA preparation was checked spectrophotometrically by measuring A260/A280 ratio as well as checked for integrity on a formaldehyde-MOPS gel. An A260/A280 value between 1.8 and 2.0 suggested that the RNA was intact and pure. Finally, the total RNA in the samples was estimated by measuring A260. Poly (A⁺) mRNA was isolated by affinity chromatography on oligo (dT)-cellulose as described by Sambrook *et al.* (1989).

### EXAMPLE 2

### cDNA library construction

cDNA prepared from poly (A+) mRNA using Oligo-dT columns was size fractionated over SizeSep-400 spun column and directionally cloned in the *Sal* I (5') /*Not* I (3') sites of pSPORT1. The cDNA library was constructed using SuperScript II Reverse Transcriptase and primer-adapters for *SalI* and *NolI* enzyme sites enabling cDNA inserts to be directionally cloned in plasmid vectors well known in art. The vector pSPORT1 was utilized for cloning cDNA fragments. The common methods for cDNA synthesis involve using poly (A⁺) RNA as a template for reverse transcription employing an oligo (dT) primer and a reverse transcriptase enzyme to synthesize first strand cDNA. These methods for synthesis of cDNA and cloning in suitable vectors are well known in art. The ligated cDNA library was transformed into *E. coli* DH5-α strain. *E. coli* transformation was carried out by the method well known in the art. A library of approximately 10⁵ recombinants was obtained (Parani M, 1999). Plasmid DNA from approximately (∼1800) randomly selected clones was extracted by alkaline lysis (Feliciello and Chinali 1993). The DNA sequence of the selected clones was determined by single pass sequencing of the 5' end using M13 reverse primer and the BigDye Terminator method (ABI Prism 310 DNA sequencer, Applied Biosystems) to generate expressed sequenced tags (ESTs). 10-12 ESTs were randomly selected and analysed for expression (Northern) under salinity stress conditions (0.5M NaCl) in *Avicennia marina* One of these genes was found to be up-regulated in both leaves and roots *of Avicennia marina* under salinity stress. The clone was designated as Am244 and was used for further analysis. As a result of the sequence determination of the full length Am244 gene (SEQ ID NO: 1) it was found that cDNA was 600 bp in length.

### EXAMPLE 3

### Expression of Am244 gene in response to diverse abiotic stresses

### NaCl stress

One-month-old *A. marina* seedlings were conditioned for 72 hours in 0.5X MS nutrient solution with the roots dipping in the solution. Subsequently, plants were stressed with 0.5X MS containing 0.5M NaCl and leaf and root tissue frozen at 6, 12, 24 and 48 hours NaCl treatment and 12 and 24 hours after salt withdrawal. Leaf tissue was harvested and total RNA was isolated according to the method given by Chomczynski and Sacchi, 1987. The total RNA was then used for northern analysis.

Total RNA was isolated as mentioned before. Equal amounts of total RNA (30µg) were electrophoresed on a 1.5 % MOPS-formaldehyde gel, transferred to nylon membrane (Hybond-N, Amersham) and fixed by UV cross linking according to the manufacturers instructions. PCR amplified product Am244 was labeled by the random primer method (Rediprime, Amersham) using α³²P-dCTP and used as probe. Radio-labeled probe were denatured and hybridized to the membrane at 65°C in an aqueous buffer (5X SSC, 5% dextran sulphate, 0.05M Na-phosphate pH 7.2, 5X Denhardt's solution, 0.0025M EDTA, 0.4% SDS and 100 µg/ml salmon sperm DNA) for 12-16 h at 65°C and washed for 15 min each with 2X SSC, 0.1% SDS and 1X SSC, 0.1%SDS. Hybridization signals were observed on Kodak X ray films after 2-3 days of exposure.

In *A. marina* roots, Am244 was rapidly upregulated, peaking at 6 hours of salt stress and being sustained thereafter up to 48 hours; upon withdrawal of the salt stress down regulation was also rapid. In the leaf however, the induction was more gradual and reached a peak at 48 hours of NaCl treatment; salt stress withdrawal did not bring about such a dramatic drop in the transcript level as was seen in the root. Fig 1a shows up regulation of Am244 in response to salt stress in roots and leaves of *A. marina.* Am244 was then fully sequenced from both ends with universal M13F and M13R primers using the same BigDye terminator method mentioned above. The compiled Am244 sequence was then compared with those in the public database using BLASTX.

In one another experiment two month old *A. marina* seedlings were acclimatized in half strength MS salts for 2-3 days. Subsequently the seedlings were shifted to half strength MS salts supplemented with 500 mM NaCl. Leaf and root tissues were harvested prior to giving the stress (0 time point) and subsequently at intervals of 10', 20', 30', 60', two four and six hours after the application of NaCl stress. This stress application, unlike the previous one, was for a shorter time period. Total RNA was isolated as described previously. Northern blotting has been mentioned above.

In the leaf tissue of *A. marina* seedlings, there was a marginal induction of Am244 transcript seen at 20' of NaCl application which increased at 30' and was sustained at 60'. At 2 hours of NaCl application, the up-regulation of the Am244 transcript was four fold which was sustained up to 6 hours of stress examined. In the root tissue, the basal levels of expression of the Am244 transcript were higher than in leaf tissue. Upon application of stress, a two fold induction of the transcript was seen at one hour of stress that was sustained up to six hours (see Fig 1b).

### Abscisic Acid stress

For studying the effect of abscisic acid (ABA) treatment, one-month-old *A. marina* seedlings were acclimatized for 72 hours in 0.5X MS nutrient solution with the roots dipping in the solution. Subsequently, plants were treated with 0.5X MS containing 100 µM ABA and leaf and root tissue frozen at 6, 12, 24, 48 hours NaCl treatment and 12 and 24 hours after salt withdrawal. This tissue was similarly used for RNA isolation and subsequent Northern analysis.

As with salt stress, ABA treatment induced the Am244 gradually in the leaf tissue upon with maximum levels of transcript being observed at 48 hours of treatment. The expression level of the transcript dropped gradually with ABA withdrawal. In the roots, ABA treatment brought about negligible changes in Am244 expression. Fig 1a shows expression profile of Am244 in response to ABA application in roots and leaves of *A. marina*

### KCl stress

Two month old *A. marina* seedlings were acclimatized in half strength MS salts for 2-3 days. Subsequently the seedlings were shifted to half strength MS salts supplemented with 500 mM KCl. Leaf and root tissues were harvested prior to giving the stress (0 time point) and subsequently at intervals of 6, 10 and 24 hours after KCl treatment. Total RNA was isolated as described previously. Northern blotting has been mentioned before.

In the leaf tissue of *A. marina* seedlings, there was a two-fold induction of the Am244 transcript at six hours of KCl treatment that was sustained up to 24 hours. In the root tissue, there was a one-fold induction of the transcript at 10 hours of KCI treatment (Fig 1b).

### Mannitol stress

Two month old *A. marina* seedlings were acclimatized in half strength MS salts for 2-3 days. Subsequently the seedlings were shifted to half strength MS salts supplemented with 800 mM mannitol. Leaf and root tissues were harvested prior to giving the stress (0 time point) and subsequently at intervals of 6, 12 and 24 hours after mannitol treatment. Thereafter the seedlings were removed from the mannitol-containing medium and transferred to half strength MS salts only. Leaves and roots were harvested at 12 and 24 hours of mannitol withdrawal. Total RNA was isolated as described previously. Northern blotting has been mentioned before.

In the leaf tissue of *A*. *marina* seedlings, there was a three-fold induction of the Am244 transcript at 12 hours of mannitol treatment. Upon withdrawal of mannitol from the medium, transcript levels were seen to decline but not to basal levels (i.e. prior to mannitol application). In the root tissue, mannitol treatment led to a two-fold increase in the Am244 transcript that was maintained at 24 hours. Upon withdrawal of the mannitol from the medium, the transcript levels dropped below basal levels (Fig 1b).

In all the experiments carried out for studying the regulation of Am244 gene in various abiotic stress conditions in *A. marina* the Am244 gene was found to be up-regulated.

### Example 4

### in silico sequence analysis of Am244 cDNA

The Am244 cDNA sequence was subjected to BLASTX comparisons with the non-redundant protein database at the NCBI website (Altschul et al. 1990) to look for similarities for reported proteins. Protein translations from the nucleotide query sequence followed by amino acid sequence alignments for deduced protein against protein database is performed using BLASTX. The BLASTX algorithm uses the BLAST algorithm to compare the six-frame conceptual translation products of a nucleotide query sequence (both strands) against a protein sequence database. Default parameters of the program were used in all cases. A minimum *P* cutoff value of 10⁻³ (the probability that alignment would be generated randomly is 1<1000) was used to determine homology of Am244 to known proteins. The full length Am244 cDNA (SEQ ID NO: 1) sequence was analyzed for the presence of complete ORF using the ORF Finder program at the NCBI website. An ORF of 57 amino acids (SEQ ID NO: 3) was identified in the Am244 cDNA. The Am244 ORF sequence is shown in SEQ ID NO: 2. This translated Am244 ORF was subjected to PSORT analysis to determine the presence of sorting signals within it (Nakai and Horton, 1999). Similarly, HMMTOP analysis was undertaken to identify the presence of putative transmembrane segments (Tusnády and Simon, 1998). A search through the protein databases showed that homologs of the Am244 gene could be found in different groups including plants, animal fungi and bacteria, suggesting a common ancestor and a high degree of conservation through evolution. Am244 exhibits extensive similarity to proteins from Arabidopsis (RCI2A and RCI2B), barley (BLt101), wheat grass Lophopyrum (ESI3) and to predicted proteins from bacteria (YqaE), fungi (Ricl), nematodes (T23F23) and yeast (PmP3/SnA1) and belongs to the uncharacterized upf0057 family of putative plasma membrane proteins. CLUSTALW was utilized for generating multiple sequence alignment of both the full length Am244 cDNA sequence as well as the translated Am244 ORF along with its orthologs retrieved from the database to identify conserved and divergent blocks (Higgins D et al., 1994). As a result of the sequence determination of the full length Am244 gene (SEQ ID NO: 1) it was found that cDNA was 600 bp in length encoding a protein consisting of 57 amino acids (SEQ ID NO: 3). Four GATTT repeats are present in the 3' untranslated region of the Am244 cDNA. PSORT analysis of Am244 gene product indicated putative plasma membrane localization and an uncleavable N-terminus signal peptide. The presence of the two transmembrane domains was predicted by HMMTOP.

### Example 5

### Construction of recombinant plant transformation vectors

### Construction pGFP-Ala-Am244-Cl and pGFP-Ala-Am244-U1

GFP along with a flexible (Alanine)₁₀ linker was fused to the N-terminus of the Am244 cDNA fragment as shown in SEQ ID No: 2 by Splice Overlap Extension (SOEing). The use of the flexible (Alanine)₁₀ linker was based on the design by Cutler et al., 2000. The primers used for SOEing are as follows: Ala-SOE FWD (SEQ ID NO: 4), Ala-SOE REV (SEQ ID NO: 5), GFP FWD (SEQ ID NO: 6), GFP REV (SEQ ID NO: 7) and Am244 FUSION REV (SEQ ID NO: 8). The nucleotide sequence of the primers are given below.
5' GCTGCCGCAGCTGCAGCCGCTATGGCTGAAGGGACAGCAACT 3' SEQ ID NO: 4
5' AGCGGCTGCAGCTGCGGCAGCTGCGGCTGCTTTGTATAGTTCATCCATGCCATG 3' SEQ ID NO: 5
5' CTAGTCTAGAATGAGTAAAGGAGAAGAACTTTTCA 3' SEQ ID NO: 6
5' CCGCTCGAGTTATTTGTATAGTTCATCCATGCCATG 3' SEQ ID NO: 7
5' CGC GGA TCC TCA GTC CCT GGT GAT GGC C 3' SEQ ID NO: 8

All primers were diluted to a 100 ng/µl stock. Am244 cDNA fragment as shown in SEQ ID NO: 1 was cloned in the *Pst*I and *Kpn*I sites of pBluescript SK II. The construct thus derived was called pBSSK II-Am244. The clone for GFP (mGFP6) was obtained as part of a plasmid pMDC83 (Curtis M et al., 2003). The GFP fragment was digested with *Sac*I and *Kpn*I and the 0.75 Kb fragment cloned in the same sites in pBluescript SK II. The construct thus derived was called as pBSSK II-GFP. The pBSSK II-Am244 and pBSSK II-GFP clones were diluted to 1ng/µl and were used as templates for amplification prior to SOEing.
I) Ala-SOE FWD and Am244 FUSION REV were used to amplify the Am244 cDNA fragment as shown in SEQ ID NO: 1 with the following reaction conditions: Reaction volume 50 µl; 1mM total dNTPs, Am244 in PBSSK II (template) 10 ng/µl; Ala-SOE FWD 150 ng; Am244 FUSION REV 100 ng, 2.5U Pfu Turbo™ (Stratagene) in 1X Pfu Turbo buffer. PCR parameters were as follows: 94°C for 3' (pre-amplification denaturation); 18 PCR cycles of 94°C for 1', 59°C for 1', 68°C for 2'; Final extension at 68°C for 7'.
II) Ala-SOE REV and GFP FWD were used to amplify GFP with the following reaction conditions: Reaction volume 50 µl; 1mM total dNTPs, GFP in PBSSK II (template) 10 ng/µl; Ala-SOE REV 150 ng; GFP FWD 100 ng, 2.5U Pfu in 1X Pfu buffer. PCR parameters: 94° C for 3' (pre-amplification denaturation); 18 PCR cycles of 94° C for 1', 59° C for 1', 68° C for 2'; Final extension at 6 ° C for 7'.

Amplification (I) gave a band size of about 170 bp while amplification (II) gave a band size of about 720 bp. The amplification products were gel eluted and quantified. (I/Am244) and (II/GFP) were mixed in a 4.2:1.0 molar ratio (10ng: 2.5 ng), heated to 68°C for 2' and chilled. This annealed product was used as template to amplify the GFP-(Alanine)₁₀-Am244 fragment. The reaction conditions for this were as follows: Reaction volume 50 µl; 1mM total dNTPs, amplification (I/Am244) and amplification (II/GFP) products in a molar ratio of 4.2: 1.0 (2.5 ng: 10 ng; GFP FWD 125 ng; Am244 FUSION REV 100 ng, 2.5U Pfu in 1X Pfu buffer. PCR parameters: 94°C for 3' (pre-amplification denaturation); 18 PCR cycles of 94°C for 1', 59°C for 1', 68°C for 2'; Final extension at 68°C for 7'. Finally, a fragment of about 900 bp was obtained wherein 3' GFP-(Alanine)₁₀ was fused to 5' end of Am244 cDNA fragment as shown in SEQ ID NO: 2.

The fused fragment was gel eluted and cloned in *Sma*I site of pBSSK II to give rise to the plasmid called pBSSK II-GFP-Ala₁₀-Am244 The sequence of the GFP-(Alanine)₁₀-Am244 ORF was verified by sequencing with conventional primers such as M13F and M13R primers and also the primers Am244 FUSION REV, GFP FWD and GFP REV. The reads were subsequently compiled and checked for an intact ORF using translation tools available at EXPASY (www. expasy.com; Bairoch A et al, 2003). Following this, the 921 bp band was excised from pBSSK 11 using the restriction enzymes *Bam* HI and *Xba* I and cloned in the pCAMBIA 1301+35S also digested with *Bam* HI and *Xha* I. These restriction sites were introduced in the Am244 FUSION REV and GFP FWD primers, respectively. The pCAMBIA 1301+35S is a modified binary plasmid vector where the CaMV 35S promoter is cloned in the *Hind* III and *Xba* I sites (multiple cloning site) of the binary plasmid vector pCAMBIA 1301 (Hajdukiewicz et al., 1994). The orientation of the GFP-(Alanine)₁₀-Am244 ORF in the pCAMBIA 1301+35S is shown in Fig 2 and the construct has been named pGFP-Ala-Am244-C1.

Another construct designated as pGFP-Ala-Am244-U1 comprises of pCAMBIA 1301+ Ubiquitin and the GFP-(Alanine)₁₀-Am244 ORF sequence cloned downstream of the ubiquitin promoter.

### Construction of plant transformation vectors pAm244-C1, pAm244-C2, pAm244-U1, and pAm244-U2

Different plasmid vectors were constructed by cloning Am244 cDNA fragment as shown in SEQ ID NO: 1 or 2 in a modified binary vectors pCAMBIA 1301+CaMV35S to form pAm244-C1 and pAm244-C2 respectively. Similarly recombinant vectors pAm244-U1 and pAm244-U2 were constructed by cloning Am244 cDNA fragment as set forth in SEQ ID NO: 1 or 2 in modified binary vector pCAMBIA 1301+ubiquitin downstream of Ubiquitin promoter. The recombinant vector thus constructed were mobilized in the *Agrobacterium* cells by the freeze thaw method (Holsters M D et al., 1978) and used for the plant transformation.

### Tobacco Transformation with pGFP-Ala-Am244-C1

*Agrobacterium-*mediated transformation of tobacco (*Nicotiana tabacum*) cv. Wisconsin was carried out by the standard protocol (Horsch RB et al., 1985). Briefly, sterile tobacco leaf discs were cut and transferred to Murashige and Skoog (MS) medium containing 3% sucrose, 1mg/L BAP, 1mg/L NAA, 0.8 % Bacto-Agar, pH 5.6 at 28 °C in 16 hours light and 8 hours darkness for 24 hours prior to transformation. 100 ml of an overnight grown culture of pGFP-Ala-Am244-C1 transformed *Agrobacterium* strain was resuspended in 0.5X MS liquid medium with 3% sucrose, pH5.6 (5 ml). The leaf discs were subsequently co-cultivated with the *Agrobacterium* (transformed with pGFP-Ala-Am244-C1) for 30 minutes. The discs were dried on sterile No. 1 Whatmann discs and transferred to MS medium containing 3% sucrose, 1mg/L Benzylaminopurine (BAP), 1mg/L Napthaleneaceticacid (NAA), 0.8 % Bacto-Agar, and pH 5.6 at 28°C in 16 hours light and 8 hours darkness for 48 hrs. The leaf discs were given several washes in half strength liquid MS medium with 1.5 % sucrose, pH 5.6 containing 250 mg/mL cefotaxime. Excess moisture on the leaf discs was blotted on sterile Whatmann No. I filter paper. The discs were then placed on selection media, that is, MS medium containing 3% sucrose, 1mg/L BAP, 1mg/L NAA, 0.8 % Bacto-Agar, pH 5.6 containing 250 mg/mL cefotaxime and 25mg/L hygromycin at 28°C in 16 hours light and 8 hours darkness. The leaf discs were transferred to fresh selection media every 14 days until multiple shoot regeneration was seen. Shoot regeneration was seen between 20-45 days after first placing on the selection media. Regenerated independent shoots were then transferred to rooting medium (MS medium containing 3% sucrose, 0.8 % Bacto-Agar, pH 5.6 containing 250 mg/mL cefotaxime and 25mg/L hygromycin at 28°C in 16 hours light and 8 hours darkness). After establishment of roots in the medium, the plants were transferred to fresh rooting medium every one month, each time transferring a shoot cut from the previous plant. Transformation of plants was confirmed by β-glucouronidase (GUS) staining of stem, leaf and root sections of the plant. The protocol for GUS staining was according to Jefferson RA et al. 1987. GUS positive plants comprising the for the pGFP-Ala-Am244-C1 T-DNA were then screened for GFP fluorescence using the Nikon Epifluorescence microscope.

### Example 6

### Epitope tagging of the Am244 cDNA

The Am244 cDNA as shown in SEQ ID No. 1 cloned in the HindIII and PstI sites of pBSSK II (pBSSK-Am244) was used as template for epitope tag insertion. Two partially overlapping and complementary primers were designed to introduce the c-myc tag sequence in the middle of the Am244 cDNA and at the N-terminus of the Am244 ORF without disturbing the Am244 5' UTR. The two primers used are Am244-Mut-Fwd (SEQ ID NO: 9) and Am244-Mut-Rev (SEQ ID NO: 10). Nucleotide sequence of these primers is given below.
5'GAACAAAAGTTGATTTCTGAAGAAGATCTGATGGCTGAAGGGACAGCAACTTGTATCGATATTG3' SEQ ID NO: 9
5'CAGATCTTCTTCAGAAATCAACTTTTGTTCCATTTTTGCCTTCCCTTGTTTGATTTTACCAAGAC3' SEQ ID NO: 10

Am244-Mut-Fwd (SEQ ID NO: 9) codes for part of the c-myc tag (marked in bold) and the beginning of the Am244 ORF. Am244-Mut-Rev (SEQ ID NO: 10) codes for the entire c-myc tag (marked in bold) as well as part of the Am244 5' UTR.

### PCR conditions

The insertion of the c-myc tag sequence within the Am244 cDNA was achieved according to Wang and Malcolm. Briefly, two single primer PCR reactions are carried out generating 'hybrid' linearized plasmids (with one wild type and one newly generated mutagenised strand). These linearized hybrid plasmids are then mixed and used as templates in the subsequent PCR steps to obtain hemi-methylated DNA as well as newly synthesized mutagenized non-methylated DNA. The parent and hemi-methylated DNA are removed by DpnI digestion while the newly synthesized mutagenized non-methylated DNA remains undigested. The reaction conditions employed were as follows: Two separate single primer reactions (25 µl each) were set up, one with the Am244-Mut-Fwd primer and the other with the Am244-Mut-Rev primer. The following components were mixed together template DNA (pBSSK-Am244) - 50 ng; dNTPs - 250 µM; 10X PCR buffer - 2.5µl, Am244-Mut-Fwd or Rev primers - 12 pmoles and heated to 95°C for 3'. 1.25 U of Pfu Polymerase was added and four PCR cycles were carried out under the following conditions: 95°C - 30s; 55°C -1'; 68°C - 8'. The two single primer reactions were mixed and allowed to proceed for 16 more cycles under the following conditions: 95°C - 30s; 55°C - 1'; 68°C - 8'. 1µl of Dpnl was added to the reaction mix and held at 37°C for 90'. 5µl of the PCR reaction mix was used for transformation into ultracompetent XL-2 Blue cells. Seven colonies were obtained and were 'size' screened for the c-myc insertion using the universal M13R (SEQ ID NO: 11) and Am244 REV1 primer (SEQ ID NO: 12)
5' AGCGGATAACATTTCACACA GG 3' SEQ ID NO: 11
5' GATACAAGTTGCTGTCCCTTG 3' SEQ ID NO: 12

PCR reaction conditions: 95°C - 3'; 95°C - 30s, 57°C - 30s; 72°C - 30s (30 cycles); 72°C - 7'; 4°C - ∞. All seven colonies screened showed a larger fragment size (with a 33 bp insertion) as compared to the PCR-generated fragment obtained from pBSSK-Am244. Plasmids were prepared from all the seven colonies and sequenced with the M13R and M13F primers using terminator method. The 33 nucleotide insertion was found at the N-terminus of the Am244 ORF. This plasmid is referred to as pBSSK-mycAm244. pBSSK-mycAm244 was digested with BamHI and the excised myc-tagged Am244 cDNA cloned in the modified binary plasmid vector pCAMBIA1301+35S (where the CaMV 35S promoter was cloned in the *Hind* III and *Xba* I sites in the multiple cloning sites of the binary plasmid vector pCAMBIA 1301). The orientation of the myc-tagged Am244 cDNA in the plasmid vector pCAMBIA1301+35S is shown in FIG 3 and the construct has been named pMyc-Am244-C1.

Similarly other recombinant transformation vector comprising c-myc tag sequence in the middle of the Am244 cDNA (SEQ ID NO: 1) operably linked to ubiquitin promoter. The recombinant vector thus constructed was designated as pMyc-Am244-U1.

Another recombinant vector comprising c-myc tag sequence at the N-terminus of the Am244 (SEQ ID NO: 2) operably linked to CaMV 35S promoter. The recombinant vector thus constructed was designated as pMyc-Am244-C2.

Another recombinant vector comprising c-myc tag sequence at the N-terminus of the Am244 (SEQ ID NO: 2) operably linked to ubiquitin promoter. The recombinant vector thus constructed was designated as pMyc-Am244-U2.

### Example 7

### Tobacco Transformation with pMyc-Am244-C1

Various recombinant plant transformation vectors describe as above were mobilizes into *Agrobacterium* strain LBA 4404 and EHA 105 and used for tobacco and rice transformation. The pMyc-Am244-C1 construct was mobilized into *Agrobacterium tumefaciens* strain LBA4404 by the freeze-thaw method.

*Agrobacterium*-mediated transformation of tobacco (*Nicotiana tabacum*) cv. Petit Havana was carried out by the standard protocol. Briefly, sterile tobacco leaf discs were cut and transferred to Murashige and Skoog (MS) medium containing 3 % sucrose, 1mg/L BAP, 1mg/L NAA, 0.8 % Bacto-Agar, pH 5.6 at 28°C in 16 hours light and 8 hours darkness for 24 hours prior to transformation. 100 ml of an overnight grown culture of pGrP-Ala-Am244 transformed *Agrobacterium* strain was resuspended in 0.5X MS liquid medium with 3 % sucrose, pH 5.6 (5 ml). The leaf discs were subsequently co-cultivated with the resuspended pMyc-Am244 transformed *Agrobacterium* for 30 minutes. The discs were dried on sterile No. 1 Whatmann discs and transferred to MS medium containing 3 % sucrose, 1mg/L BAP, 1mg/L NAA, 0.8 % Bacto-Agar, pH 5.6 at 28°C in 16 hours light and 8 hours darkness for 48 hrs. The leaf discs were given several washes in liquid MS medium with 3 % sucrose, pH 5.6 containing 250 mg/mL cefotaxime. Excess moisture on the leaf discs was blotted on sterile Whatmann No. 1 filter paper. The discs were then placed on selection media, that is, MS medium containing 3% sucrose, 1mg/L BAP, 1mg/L NAA, 0.8 % Bacto-Agar, pH 5.6 containing 250 mg/mL cefotaxime and 25mg/L hygromycin at 28°C in 16 hours light and 8 hours darkness. The leaf discs were transferred to fresh selection media every 14 days until multiple shoot regeneration was seen. Shoot regeneration was seen between 20-35 days after first placing on the selection media. Regenerated independent shoots were then transferred to rooting medium (MS medium containing 3 % sucrose, 0.8 % Bacto-Agar, pH 5.6 containing 250 mg/mL cefotaxime and 25 mg/L hygromycin at 28°C in 16 hours light and 8 hours darkness). After establishment of roots in the medium the plants transferred to fresh rooting medium every 14 days, each time transferring a shoot cut from the previous plant. Transformation of plants was confirmed by β-glucouronidase (GUS) staining of stem, leaf and root sections of the plant. The protocol for GUS staining was according to Jefferson RA *et al,* 1987. Twenty GUS positive lines were obtained from 23 independently transformed regenerants obtained. Leaves were harvested from these plants for genomic DNA isolation.

### Isolation of Genomic DNA

Genomic DNA from the transformed tobacco plant was isolated using the protocol of Richards EJ (1987). 2-3 grams of tobacco leaves (harvested from sixteen Myc-Am244 transformed lines as well as control untransformed tobacco cv. Petit Havana) were ground to a fine powder using liquid nitrogen in a mortar and pestle. The ground tissue was suspended in 8-10 ml of CTAB buffer (2% cetyltrimethylammonium bromide, 100mM Tris-Cl pH 8.0, 20mM EDTA, 1.4M NaCl and 0.2% β-mercaptoethanol) and incubated in a water bath at 65°C for 30 minutes in centrifuge tubes. An equal volume of choloroform: isoamyl alcohol (24:1) was added and after inversion the tubes centrifuged at 10,000 rpm for 15°C to allow for phase separation. The upper aqueous phase was transferred to a new tube and 0.6 volumes of isopropanol were added. The samples were left overnight at -20°C for DNA precipitation. The tubes were then centrifuged at 12,000 rpm to pellet the DNA and the supernatant decanted. The pellet was allowed to air dry and resuspended in TE buffer (0.5 ml). The resuspended sample was treated with RNAse A (10µg/ml) and incubated at 37°C for two hours. An equal of phenol: choloroform (1:1) was added, mixed by inversion and centrifuged at 10,000 rpm for 10' to separate the phases. The upper aqueous phase was transferred to a new Eppendorf and an equal volume of chloroform added, mixed by inversion and centrifuged at 10,000 rpm for 10' to separate the phases. The upper aqueous phase was transferred to a new Eppendorf and 0.1 volume of 3M sodium acetate pH 5.2 was added followed by 2.5 volumes of absolute ethanol and kept for precipitation overnight at -20°C. The samples were then centrifuged at 12,000 rpm to pellet the genomic DNA and the supernatant discarded. The pellet was rinsed with 70 % ethanol and centrifuged at 12,000 rpm, the wash discarded and allowed to air dry. The genomic DNA pellet was resuspended in TE.

PCR analysis was carried out with gene specific primers for confirming the presence of Am244 DNA in transgenic plants.

### Southern Hybridization

The genomic DNA (40µg) isolated from sixteen lines Myc-Am244 lines and control (untransformed tobacco cv. Petit Havana) was digested with HindIII overnight. The digested genomic DNA was loaded on a 0.8% agarose gel (1X TBE) and run at 35 V for 16 hours. The gel was photographed and transferred to nylon membrane (Hybond N+, Amersham) using the alkaline transfer method mentioned in Sambrook et al. Briefly, the gel was incubated in denaturation solution (0.4N NaOH, 1M NaCl) for 30'. The capillary transfer method was used to transfer the genomic DNA to nylon membrane (Hybond N+, Amersham). Following transfer for 14-16 hours, the nylon membrane was rinsed twice with neutralization buffer (0.5M Tris-Cl pH 8.0, 1 M NaCl) for 30'. The DNA was then cross-linked to the nylon blot using a UV-crosslinker (Hoefer, UVC500). Pre-hybridization of the Southern blots was carried out at 56°C in the phosphate hybridization buffer (0.5M Na₂HPO₄, 7 % SDS and 1 mM EDTA @ 150µl of buffer/cm² of the membrane) for 2 hours in a hybridization chamber (Hybridization Incubator Combi-H, Finemould Precision Inc.). 100 ng of PCR amplified and gel-purified Am244 cDNA was labeled in the presence of 5µl, of α-³²P-dCTP (BRIT, 3500 Ci/mmol) using the RediPrime Kit (Amersham) as per the manufacturer's instructions. The α-³²P-dCTP was purified through a Sephadex G-50 column to remove un-incorporated nucleotides. The probe was denatured for 5-7' in boiling water, chilled on ice and added to the blot with fresh phosphate hybridization buffer (@ 150µl of buffer/cm² of the membrane) and incubated at 56°C for 14-16 hours in the hybridization oven. The membrane was washed with 2X SSC, 0.1% SDS for 15' and exposed to the PhosphorImager screen (Personal Molecular Imager, BioRad) for 14-16 hours. The exposed images were scanned and analysed using the Quantity One software (BioRad).

The pMyc-Am244-C1 T-DNA has a single HindIII site flanking the 35S CaMV promoter driving the expression of myc-tagged Am244 cDNA. Digestion of the genomic DNA obtained from GUS positive Myc-Am244 transformed lines with HindIII would thus help in the identification of single copy insertions of the pMyc-Am244 T-DNA in the tobacco plants examined. Southern analysis of Myc-Am244 transformed lines revealed that there were single copy insertions of the Myc-Am244. cDNA in Lines 1, 6, 7, 8, 11 and 17.

### Example 8

### Transformation of Rice using Agrobacterium mediated method

Rice calli were transformed with the recombinant vectors disclosed in the invention by *Agrobacterium*-transformation methods as described in Example 5 and Example 7. Other standard protocols which are well known to the person skilled in art can also be used.

Rice calli was generated from mature seed scutella of pusa basmati 1 on a callus induction medium (MS inorganic + MS vitamins + 2,4D (2mg/ml)). Three week old calli were then infected with the *Agrobacterium tumefaciens* LBA4404 carrying any one of the recombinant vectors as described above such as pGFP-Ala-Am244-U1, pAm244-U1 pAm244-U2, pMyc-Am244-U1 or pMyc-Am244-U2. The infected calli were washed for a period of thirty minutes and then dried on a sterile filter paper. The dried calli were then transferred to a selection medium (callus induction medium containing Hygromycin (50µg/ml)) for a period of 6 weeks. The calli were sub-cultured every fifteen days. The selected calli were then transferred to the regeneration medium (MS inorganic, MS vitamins, benzyl aminopurine (BAP (1.5mg/L)), kinetin (0.5mg/L) and NAA (0.5mg/L)). The regenerants were transferred to the rooting medium (MS inorganic and MS vitamins without hormones). The plantlets were subsequently transferred to the hardening medium for a span of two weeks and finally transferred to the soil in pots to raise the next generation of seeds. The tillers of the rice plants were bagged before the onset of flowering in order to promote self-pollination. The seeds from the selfed plants were collected and again sown for the next generation. The second generation rice plants were analyzed for the presence of the Am244 DNA as shown in SEQ ID NO: 1 or 2. The transgenic rice plants were screened for the localization of the polypeptide as shown in SEQ ID NO: 3.

These plants were tested for the presence of the Am244 DNA and also for copy number of the inserted gene. Further the plants were screened for tolerance to abiotic stress. The details are given in Example 10.

### Example 9

### Microscopy and Imaging

Leaf peels mounted in water were prepared from *GUS* positive tobacco and rice plants transformed with pGFP-Ala-Am244-C1 or pGFP-Ala-Am244-U1 respectively and examined under the Nikon Optiphot-2 phase contrast microscope fitted with an Episcopic fluorescence attachment (100W Hg lamp). Fluorescence imaging of guard cells was carried out using the Nikon B2A filter set (excitation 450-490, Dichroic mirror 510, Barrier filter 520) and the Fluor 40X dry objective. Photographs were taken with a 35mm FX-35DX camera using the Microflex HfX-DX attachment for automatic exposure adjustments on a Kodak ASA 400 film.

Guard cell imaging of pGFP-Ala-Am244-C1 and pGFP-Ala-Am244-C1 transformed tobacco and rice plants respectively showed localization of the green fluorescence at the periphery of the guard cells and close to the cell wall, suggesting plasma membrane localization of Am244. Chlorophyll auto-fluorescence was used to locate the chloroplasts under the same imaging conditions. Bright field images and fluorescence images of the tobacco guard cell are enclosed in FIG 4.

### Example 10

### Expression Analysis of transgenic plant

### Whole-plant Salt stress Treatments:

The salt tolerance conferred by over-expressing Am244 gene in tobacco and rice transgenics was analyzed by performing whole plant salt stress treatments. Phenotypic growth retardation study was also performed between control and transgenic plants. Three control and transgenic plants were grown initially in ½ MS for 1 week. Later, they were transferred to ½ MS medium supplemented with 150 mM and 200 mM NaCl. It was observed that in 150 mM NaCl, transgenic plants showed better rooting when compared to control plants. At 200 mM NaCl, both control and transgenic plants did not root. It was also found that the transgenic plants suffered less damage in 150 mM and 200 mM NaCl stress. Phenotypic growth retardation was not evident in control and in both tobacco and rice transgenic plants grown in pots and irrigated with 150 mM NaCl solution for 1½ week.

Similarly experiments were conducted for analyzing the transgenic plants for other stresses.

ABA stress: A stress of 1µM ABA was given to control and transgenic plants (rice and tobacco) for same time intervals. It was observed that in 1µM ABA, transgenic plants showed better rooting when compared to control plants which confirmed overexpression of Am244 DNA in rice and tobacco.

KCI stress: Control and transgenic were supplemented with 500mM KCl. It was observed that in 500 mM KCl, transgenic plants showed better rooting when compared to control plants which confirmed overexpression of Am244 DNA in rice and tobacco.

Mannitol stress: Control and transgenic were supplemented with 800mM KCl. It was observed that in 800 mM Mannitol, transgenic plants showed better rooting when compared to control plants which confirmed overexpression of Am244 DNA in rice and tobacco.

### References

1. Altschul SF, Gish W, Miller W, Myers EW, Lipman DJ (1990) Basic local alignment search tool. J Mol Biol 215:403-410
2. Boon PI, Allaway WG (1982) Assessment of leaf-washing techniques for measuring salt secretion in Avicennia marina (Forsk.) Vierh. Aust J Plant Physiol 9:725-734
3. Capel J, Jarillo JA, Salinas J, Martinez-Zapater JM (1997) Two homologous low-temperature-inducible genes from Arabidopsis encode highly hydrophobic proteins. Plant Physiol. 115: 569-76
4. Chauhan S, Forsthoefel N, Ran Y, Quigley F, Nelson DE, Bohnert HJ (2000) Na+/myo-inositol symporters and Na+/H+-antiporter in Mesembryanthemum crystallinum. Plant J 24:511-522
5. Chomezynski P, Sacchi N (1987) Single-step method of RNA isolation by acid guanidinium thiocyanate-phenol-chloroform extraction. Anal Biochem 162:156-159
6. Curtis MD, Grossniklaus U (2003) A gateway cloning vector set for high-throughput functional analysis of genes in planta. Plant Physiol. 133: 462-9
7. Cushman JC, Bohnert HJ (2000) Genomic approaches to plant stress tolerance. Curr Opin Plant Biol 3:117-124
8. Cutler SR, Ehrhardt DW, Griffitts JS, Somerville CR (2000) Random GFP::cDNA fusions enable visualization of subcellular structures in cells of Arabidopsis at a high frequency. Proc Natl Acad Sci USA. 97: 3718-23
9. Feliciello I, Chinali G (1993) A modified alkaline lysis method for the preparation of highly purified plasmid DNA from Escherichia coli. Anal Biochem 212:394-401
10. Goddard NJ, Dunn MA, Zhang L, White AJ, Jack PL, Hughes MA (1993) Molecular analysis and spatial expression pattern of a low-temperature-specific barley gene, blt101. Plant Mol Biol. 23: 871-9
**11.** Gulick PJ, Shen W, An H (1994) ESI3, a stress-induced gene from Lophopyrum elongatum. Plant Physiol. 104:799-800
12. Hajdukiewicz P Svab Z, Maliga, P (1994) The small versatile pPZP family of Agrobacterium binary vectors for plant transformation. Plant Mol Biol 25:989-994.
13. Higgins D, Thompson J, Gibson T, Thompson JD, Higgins DG, Gibson TJ (1994) CLUSTAL W: improving the sensitivity of progressive multiple sequence alignment through sequence weighting, position-specific gap penalties and weight matrix choice. Nucleic Acids Res. 22:4673-4680.
14. Holsters M D, de Waele A, Depicker E, Messens M, Van Montagu, J Schell (1978) Transfection and transformation of A. tumefaciens. Mol. Gen. Genet. 163:181-187
15. Horsch RB, Fry JE, Hoffmann NL, Eichholtz D, Rogers S G, Fraley RT (1985) A simple and general method for transferring genes into plants. Science 227:1229-1231.
16. Jefferson RA, Kavanagh TA, Bevan MW (1987) GUS fusions: Beta-glucuronidase as a sensitive and versatile gene fusion marker in higher plants. EMBO J 6:3901-3907.
17. Munns R, Husain S, Rivelli AR, James RA, Condon AG, Lindsay MP, Lagudah ES, Schachtman DP, Hare RA (2002) Avenues for increasing salt tolerance of crops, and the role of physiologically based selection traits. Plant Soil 247:93-105
18. Nakai K, Horton P (1999) PSORT: a program for detecting sorting signals in proteins and predicting their subcellular localization. Trends Biochem Sci. 24: 34-6.
19. Parani M (1999) Isolation of salt tolerance genes from mangroves: characterization of betaine aldehyde dehydrogenase (BADH) gene from Avicennia marina and its expression in tobacco transgenic system. PhD thesis, Anna University, India
20. Rao AN (1987) Mangrove ecosystems of Asia and the Pacific. In: Umali RM, Zamora PM, Gotera RR, Jara RS, Camacho AS (eds) Mangroves of Asia and the Pacific: status and management. UNDP-UNESCO Technical Report, pp 1-41
21. Richards EJ (1987) Preparation of genomic DNA from plant tissue. In: Current Protocols in Molecular Biology, (eds. F. Ausubel et al) John Wiley and Sons, New York, pp. 2.3.1-2.3.3.
22. Sambrook, J, Fritsch EF, and Maniatis T Molecular Cloning, 2nd ed. Cold Spring Harbor Laboratory Press, Cold Spring Harbor, NY.
23. Shimony C, Fahn A, Reinhold L (1973) Ultrastructure and ion gradients in the salt glands of Avicennia marina (Forsk.) Vierh. New Phytol 72:27-36
24. Tusnády GE and Simon I (1998) Principles Governing Amino Acid Composition of Integral Membrane Proteins: Applications to Topology Prediction." J. Mol. Biol. 283: 489-506.
25. Van West P, Kamoun S, van't Klooster JW, Govers F (1999) Ric1, a Phytophthora infestans gene with homology to stress-induced genes. Curr Genet. 36: 310-5
26. Wang W, Malcolm BA. Two-Stage Polymerase Chain Reaction Protocol Allowing Introduction of Multiple Mutations, Deletions, and insertions using QuikChange™ Site-directed mutagenesis. In: In vitro Mutagenesis Protocols. 2nd ed. Series: Methods in Molecular Biology v. 182. Edited by J. Braman, Humana Press Inc. Totowa NJ
27. Yale J, Bohnert HJ (2001) Transcript expression in Saccharomyces cerevisiae at high salinity. J Biol Chem. 276: 15996-6007
28. Zhu JK (2002) Salt and drought stress signal transduction in plants. Annu Rev Plant Biol 53:247-273
29. Preeti AM, et al. (2005) Generation and analysis of expressed sequence tags from the salt-tolerant mangrove species Avicennia marina (Forsk) Vierh., Theor. Appl. Genet. 110:416-424
30. Zhou HT, Lin P. (2002) MEDLINE Abstract of Sheng Wu Gong Cheng Xue Bao (2002), 18(1):51-4

## Claims

1. Use of an isolated nucleic acid molecule for enhancing the tolerance to drought stress, salt stress and/or dehydration stress in plants wherein said isolated nucleic acid molecule has a nucleotide sequence with at least 90% homology to the nucleotide sequence set forth in SEQ ID NO: 1 or SEQ ID NO: 2, wherein said sequence codes for a polypeptide having an amino acid sequence as shown in SEQ ID NO: 3.

2. The use of claim 1, wherein said nucleic acid molecule comprises a nucleotide sequence as set forth in SEQ ID NO: 1 and SEQ ID NO: 2.

3. Use of a polypeptide having an amino acid sequence as set forth in SEQ ID NO: 3 for enhancing the tolerance to drought stress, salt stress and/or dehydration stress in plants, wherein said polypeptide is encoded by the nucleic acid as claimed in claim 1.

4. Use of an expression cassette for enhancing the tolerance to drought stress, salt stress and/or dehydration stress in plants, wherein said expression cassette comprises the nucleic acid molecule as claimed in claim 1 or claim 2 operably linked to a plant expressible regulatory sequence.

5. The use of claim 4, wherein the regulatory sequence is selected form a group consisting of CaMV 35S, NOS, OCS, AdhI, AdhII and Ubi-1.

6. Use of a DNA construct for enhancing the tolerance to drought stress, salt stress and/or dehydration stress in plants, wherein said DNA construct comprises the expression cassette of claim 4.

7. The use of claim 6, wherein said construct further comprises another expression cassette comprising a selectable marker gene operably linked to the regulatory sequence as claimed in claim 5.

8. The use of claim 7, wherein the selectable marker gene is selected from a group consisting of nptII, hptII, pat and bar.

9. The use of claim 6 or claim 7, wherein the DNA construct further comprises another expression cassette comprising a scorable marker gene operably linked to the regulatory sequence as claimed in claim 5.

10. The use of claim 9, wherein the scorable marker gene is selected from a group consisting of GUS, GFP, LUC and CAT.

11. Use of a recombinant vector for enhancing the tolerance to drought stress, salt stress and/or dehydration stress in plants, wherein said recombinant vector comprises the DNA construct of any one of claims 6-10.

12. The use of claim 11, wherein said vector is a plant transformation vector.

13. Use of a recombinant host cell for enhancing the tolerance to drought stress, salt stress and/or dehydration stress in plants, wherein said host cell comprises the recombinant vector of claim 11.

14. The use of claim 13, wherein the host cell is a prokaryotic or eukaryotic cell.

15. The use of claim 14, wherein the prokaryotic cell is either *E*. *coli* or *Agrobacterium.*

16. The use of claim 14, wherein the eukaryotic cell is a plant cell.

17. A drought stress, salt stress and/or dehydration stress tolerant transgenic plant or plant cell or plant tissue comprising an expression cassette comprising an isolated nucleic acid molecule having a nucleotide sequence with at least 90% homology to the nucleotide sequence set forth in SEQ ID NO: 1 or SEQ ID NO: 2, wherein said sequence codes for a polypeptide having an amino acid sequence as shown in SEQ ID NO: 3 operably linked to a plant expressible regulatory sequence, wherein the expression of the said nucleic acid molecule results in the enhanced tolerance to drought stress, salt stress and/or dehydration stress in said plant, plant cell and plant tissue.

18. A method of producing a drought stress, salt stress and/or dehydration stress tolerant transgenic plant, said method comprising introducing an expression cassette comprising an isolated nucleic acid molecule having a nucleotide sequence with at least 90% homology to the nucleotide sequence set forth in SEQ ID NO: 1 or SEQ ID NO: 2, wherein said sequence codes for a polypeptide having an amino acid sequence as shown in SEQ ID NO: 3 operably linked to a plant expressible regulatory sequence in a plant genome by using a transformation method, thereby producing a drought stress, salt stress and/or dehydration stress tolerant transgenic plant.

19. The method of claim 18, wherein said transformation method is selected from a group consisting of *Agrobacterium* mediated transformation, particle bombardment, vacuum-infiltration, in planta transformation and chemical methods.

20. The method of claim 19, wherein the *Agrobacterium* mediated transformation comprises:
a) obtaining suitable explants from a plant,
b) constructing the recombinant vector of claim 11,
c) mobilizing said vector in an *Agrobacterium* cell to produce a recombinant *Agrobacterium* cell,
d) co-cultivating said explants with said recombinant *Agrobacterium* cell to produce transformed plant cells,
e) culturing said transformed plant cells to produce a drought stress, salt stress and/or dehydration stress tolerant transgenic plant.

21. The method of claim 18 or 20, wherein said plant is a monocotyledonous or a dicotyledonous plant.

22. The method of claim 21, wherein the monocotyledonous plant is selected from a group consisting of rice, maize, wheat, barley and sorghum.

23. The method of claim 22, wherein said monocotyledonous plant is a rice plant.

24. The method of claim 21, wherein the dicotyledonous plant is selected from a group consisting of tobacco, tomato, pea, soybean, Brassica, chickpea and pigeon pea.

25. The method of claim 20, wherein said explants are selected from a group consisting of cotyledons, hypocotyls, leaves, anthers, callus, cotyledonary nodes, stems and roots.

## Patentansprüche

1. Verwendung eines isolierten Nukleinsäuremoleküls zur Erhöhung der Toleranz gegenüber Trockenstress, Salzstress und/oder Dehydrierungsstress in Pflanzen, wobei das isolierte Nukleinsäuremolekül eine Nukleotidsequenz mit mindestens 90% Homologie zu der in SEQ ID NR: 1 oder SEQ ID NR: 2 dargestellten Nukleotidsequenz hat, wobei die Sequenz für ein Polypeptid mit einer Aminosäuresequenz wie in SEQ ID NR: 3 dargestellt kodiert.

2. Verwendung nach Anspruch 1, wobei das Nukleinsäuremolekül eine wie in SEQ ID NR: 1 und SEQ ID NR: 2 dargestellte Nukleotidsequenz umfasst.

3. Verwendung eines Polypeptids mit einer wie in SEQ ID NR: 3 dargestellten Aminosäuresequenz zur Erhöhung der Toleranz gegenüber Trockenstress, Salzstress und/oder Dehydrierungsstress in Pflanzen, wobei das Polypeptid durch die in Anspruch 1 beanspruchte Nukleinsäure kodiert wird.

4. Verwendung einer Expressionskette zur Erhöhung der Toleranz gegenüber Trockenstress, Salzstress und/oder Dehydrierungsstress in Pflanzen, wobei die Expressionskassette das wie in Anspruch 1 oder 2 beanspruchte Nukleinsäuremolekül umfasst, welches funktionell mit einer in Pflanzen exprimierbaren regulatorischen Sequenz verknüpft ist.

5. Verwendung nach Anspruch 4, wobei die regulatorische Sequenz ausgewählt ist aus der Gruppe bestehend aus CaMV 35S, NOS, OCS, AdhI, AdhII und Ubi-1.

6. Verwendung eines DNA-Konstrukts zur Erhöhung der Toleranz gegenüber Trockenstress, Salzstress und/oder Dehydrierungsstress in Pflanzen, wobei das DNA-Konstrukt die Expressionskassette nach Anspruch 4 umfasst.

7. Verwendung nach Anspruch 6, wobei das Konstrukt zusätzlich eine andere Expressionskassette umfasst, welche ein selectable Markergen funktionell verknüpft mit der wie in Anspruch 5 beanspruchten regulatorischen Sequenz umfasst.

8. Verwendung nach Anspruch 7, wobei das selectable Markergen ausgewählt ist aus der Gruppe bestehend aus nptII, hptII, pat und bar.

9. Verwendung nach Anspruch 6 oder 7, wobei das DNA-Konstrukt zusätzlich eine andere Expressionskassette umfasst, welche ein scorable Markergen funktionell verknüpft mit der wie in Anspruch 5 beanspruchten regulatorischen Sequenz umfasst.

10. Verwendung nach Anspruch 9, wobei das scorable Markergen ausgewählt ist aus der Gruppe bestehend aus GUS, GFP, LUC und CAT.

11. Verwendung eines rekombinanten Vektors zur Erhöhung der Toleranz gegenüber Trockenstress, Salzstress und/oder Dehydrierungsstress in Pflanzen, wobei der rekombinante Vektor das DNA-Konstrukt nach einem der Ansprüche 6 bis 10 umfasst.

12. Verwendung nach Anspruch 11, wobei der Vektor ein Pflanzentransformationsvektor ist.

13. Verwendung einer rekombinanten Wirtszelle zur Erhöhung der Toleranz gegenüber Trockenstress, Salzstress und/oder Dehydrierungsstress in Pflanzen, wobei die Wirtszelle den rekombinanten Vektor nach Anspruch 11 umfasst.

14. Verwendung nach Anspruch 13, wobei die Wirtszelle eine prokaryotische oder eukaryotische Zelle ist.

15. Verwendung nach Anspruch 14, wobei die prokaryotische Zelle entweder *E. coli* oder *Agrobacterium* ist.

16. Verwendung nach Anspruch 14, wobei die eukaryotische Zelle eine Pflanzenzelle ist.

17. Ein/e Trockenheitsstress-, Salzstress- und/oder Dehydrierungsstress-tolerante transgene Pflanze oder Pflanzenzelle oder Pflanzengewebe umfassend eine Expressionskassette, umfassend ein isoliertes Nukleinsäuremolekül mit einer Nukleotidsequenz mit mindestens 90% Homologie zu der in SEQ ID NR: 1 oder SEQ ID NR: 2 dargestellten Nukleotidsequenz, wobei die Sequenz für ein Polypeptid mit einer Aminosäuresequenz wie in SEQ ID NR: 3 dargestellt kodiert, funktionell verknüpft mit einer in Pflanzen exprimierbaren regulatorischen Sequenz, wobei die Expression des Nukleinsäuremoleküls zu einer erhöhten Toleranz gegenüber Trockenstress, Salzstress und/oder Dehydrierungsstress in der Pflanze, der Pflanzenzelle und dem Pflanzengewebe führt.

18. Verfahren zur Herstellung einer Trockenstress-, Salzstress- und/oder Dehydrierungsstress-toleranten transgenen Pflanze, welches das Einführen einer Expressionskassette umfassend ein isoliertes Nukleinsäuremolekül mit einer Nukleotidsequenz mit mindestens 90% Homologie zu der in SEQ ID NR: 1 oder SEQ ID NR: 2 dargestellten Nukleotidsequenz, wobei die Sequenz für ein Polypeptid mit einer Aminosäuresequenz wie in SEQ ID NR: 3 dargestellt kodiert, funktionell verknüpft mit einer in Pflanzen exprimierbaren regulatorischen Sequenz in ein Pflanzengenom unter Verwendung eines Transformationsverfahrens umfasst, wodurch eine Trockenstress-, Salzstress- und/oder Dehydrierungsstress-tolerante transgene Pflanze hergestellt wird.

19. Verfahren nach Anspruch 18, wobei das Transformationsverfahren ausgewählt ist aus der Gruppe bestehend aus *Agrobacterium*-vermittelte Transformation, Teilchenbeschuss, Vakuuminfiltration, *in planta* Transformation und chemische Verfahren.

20. Verfahren nach Anspruch 19, wobei die *Agrobacterium* vermittelte Transformation umfasst:
a) Gewinnen eines geeigneten Explantats von einer Pflanze,
b) Herstellen des rekombinanten Vektors nach Anspruch 11,
c) Mobilisieren dieses Vektors in eine *Agrobacterium*-Zelle, um eine rekombinante *Agrobacterium*-Zelle herzustellen,
d) Co-Kultivieren der Explantate mit der rekombinanten *Agrobacterium*-Zelle, um transformierte Pflanzenzellen herzustellen,
e) Kultivieren der transformierten Pflanzenzellen, um eine Trockenstress-, Salzstress- und/oder Dehydrierungsstress-tolerante transgene Pflanze herzustellen.

21. Verfahren nach Anspruch 18 oder 20, wobei die Pflanze eine monocotyledone oder dicotyledone Pflanze ist.

22. Verfahren nach Anspruch 21, wobei die monocotyledone Pflanze ausgewählt ist aus der Gruppe bestehend aus Reis, Mais, Weizen, Gerste und Hirse.

23. Verfahren nach Anspruch 22, wobei die monocotyledone Pflanze eine Reispflanze ist.

24. Verfahren nach Anspruch 21, wobei die dicotyledone Pflanze ausgewählt ist aus der Gruppe bestehend aus Tabak, Tomate, Erbse, Sojabohne, Brassica, Kichererbse und Straucherbse.

25. Verfahren nach Anspruch 20, wobei die Explantate ausgewählt sind aus der Gruppe bestehend aus Kotyledonen, Hypokotyle, Blätter, Antheren, Callus, Knoten der Kotyledonen, Stämmen und Wurzeln.

## Revendications

1. Utilisation d'une molécule d'acide nucléique isolée pour améliorer la tolérance aux stress induits par la sécheresse, la salinité et/ou la déshydratation chez les plantes, où ladite molécule d'acide nucléique isolée présente une séquence de nucléotides ayant au moins 90 % d'homologie avec la séquence de nucléotides exprimée dans SEQ ID N° 1 ou SEQ ID N° 2, où ladite séquence code un polypeptide présentant une séquence d'acides aminés telle que présentée dans SEQ ID N° 3.

2. L'utilisation selon la revendication 1, où ladite molécule d'acide nucléique comprend une séquence de nucléotides telle qu'exprimée dans SEQ ID N° 1 et SEQ ID N°2.

3. Utilisation d'un polypeptide présentant une séquence d'acides aminés telle qu'exprimée dans SEQ ID N° 3 pour améliorer la tolérance aux stress induits par la sécheresse, la salinité et/ou la déshydratation chez les plantes, où ledit polypeptide est codé par l'acide nucléique tel qu'exposé dans la revendication 1.

4. Utilisation d'une cassette d'expression pour améliorer la tolérance aux stress induits par la sécheresse, la salinité et/ou la déshydratation chez les plantes, où ladite cassette d'expression comprend la molécule d'acide nucléique telle qu'exposée dans la revendication 1 ou la revendication 2 en liaison opératoire avec une séquence régulatrice exprimable chez les plantes.

5. L'utilisation selon la revendication 4, où la séquence régulatrice est sélectionnée dans un groupe constitué de CaMV 35S, NOS, OCS, AdhI, AdhII et Ubi-1.

6. Utilisation d'une construction d'ADN pour améliorer la tolérance aux stress induits par la sécheresse, la salinité et/ou la déshydratation chez les plantes, où ladite construction d'ADN comprend la cassette d'expression de la revendication 4.

7. L'utilisation selon la revendication 6, où ladite construction comprend en outre une autre cassette d'expression comprenant un gène marqueur sélectionnable en liaison opératoire avec la séquence régulatrice telle qu'exposée dans la revendication 5.

8. L'utilisation selon la revendication 7, où le gène marqueur sélectionnable est sélectionné dans un groupe constitué de nptII, hptII, pat et bar.

9. L'utilisation selon la revendication 6 ou la revendication 7 , où la construction d'ADN comprend en outre une autre cassette d'expression comprenant un gène marqueur détectable en liaison opératoire avec la séquence régulatrice telle qu'exposée dans la revendication 5.

10. L'utilisation de la revendication 9, où le gène marqueur détectable est sélectionné dans un groupe constitué de GUS, GFP, LUC et CAT.

11. Utilisation d'un vecteur recombinant pour améliorer la tolérance aux stress induits par la sécheresse, la salinité et/ou la déshydratation chez les plantes, où ledit vecteur recombinant comprend la construction d'ADN de l'une quelconque des revendications 6 à 10.

12. L'utilisation selon la revendication 11, où ledit vecteur est un vecteur de transformation végétale.

13. Utilisation d'une cellule hôte recombinante pour améliorer la tolérance aux stress induits par la sécheresse, la salinité et/ou la déshydratation chez les plantes, où ladite cellule hôte comprend le vecteur recombinant de la revendication 11.

14. L'utilisation selon la revendication 13, où la cellule hôte est une cellule procaryote ou eucaryote.

15. L'utilisation selon la revendication 14, où la cellule procaryote est soit *E. coli* soit *Agrobacterium.*

16. L'utilisation selon la revendication 14, où la cellule eucaryote est une cellule végétale.

17. Une plante ou cellule végétale ou tissu végétal transgénique tolérant(e) aux stress induits par la sécheresse, la salinité et/ou la déshydratation comprenant une cassette d'expression comprenant une molécule d'acide nucléique isolée présentant une séquence de nucléotides ayant au moins 90 % d'homologie avec la séquence de nucléotides exprimée dans SEQ ID N° 1 ou SEQ ID N° 2, où ladite séquence code un polypeptide présentant une séquence d'acides aminés telle que présentée dans SEQ ID N° 3 en liaison opératoire avec une séquence régulatrice exprimable chez les plantes, où l'expression de ladite molécule d'acide nucléique aboutit à la tolérance améliorée aux stress induits par la sécheresse, la salinité et/ou la déshydratation dans ladite plante, ladite cellule végétale, ledit tissu végétal.

18. Procédé de production d'une plante transgénique tolérante aux stress induits par la sécheresse, la salinité et/ou la déshydratation, ledit procédé comportant l'introduction d'une cassette d'expression comprenant une molécule d'acide nucléique isolée présentant une séquence de nucléotides ayant au moins 90 % d'homologie avec la séquence de nucléotides exprimée dans SEQ ID N° 1 ou SEQ ID N° 2, où ladite séquence code un polypeptide présentant une séquence d'acides aminés telle que présentée dans SEQ ID N° 3 en liaison opératoire avec une séquence régulatrice exprimable chez les plantes dans un génome de plante moyennant l'utilisation d'un procédé de transformation, produisant ainsi une plante transgénique tolérante aux stress induits par la sécheresse, la salinité et/ou la déshydratation.

19. Le procédé selon la revendication 18, où ledit procédé de transformation est sélectionné dans un groupe constitué de la transformation induite par *Agrobacterium,* le bombardement de particules, l'infiltration sous vide, dans les procédés de transformation végétale et chimiques.

20. Le procédé selon la revendication 19, où la transformation induite par *Agrobacterium* comprend :
a) l'obtention d'explants adéquats à partir d'une plante,
b) la construction du vecteur recombinant de la revendication 11,
c) la mobilisation dudit vecteur dans une cellule *Agrobacterium* pour produire une cellule *Agrobacterium* recombinante,
d) la co-cultivation desdits explants avec ladite cellule *Agrobacterium* pour produire des cellules végétales transformées,
e) la mise en culture desdites cellules végétales transformées pour produire une plante transgénique tolérante aux stress induits par la sécheresse, la salinité et/ou la déshydratation.

21. Le procédé selon la revendication 18 ou 20, où ladite plante est une plante monocotylédone ou dicotylédone.

22. Le procédé selon la revendication 21, où la plante monocotylédone est sélectionnée dans un groupe constitué du riz, du maïs, du blé, de l'orge et du sorgho.

23. Le procédé selon la revendication 22, où la plante monocotylédone est une plante de riz.

24. Le procédé selon la revendication 21, où la plante dicotylédone est sélectionnée dans un groupe constitué du tabac, de la tomate, du pois, du soya, de la brassicée, du pois chiche et pois cajan.

25. Le procédé selon la revendication 20, où lesdits explants sont sélectionnés dans un groupe constitué des cotylédons, des hypocotyles, des feuilles, des anthères, des calles, des noeuds cotylédonaires, des tiges et des racines.
